# EUROPEAN PATENT APPLICATION

(11) **EP 4 394 040 A1**
(43) Date of publication of application: **03.07.2024**
(21) Application number: 22881390.3
(22) Date of filing: 14.10.2022
(51) Int. Cl.: C12N 15/77, C07K 14/34, C12P 13/10

(54) **CORYNEBACTERIUM GENUS MICROORGANISM PRODUCING L-ARGININE, AND METHOD FOR PRODUCING L-ARGININE USING SAME**

(30) Priority: 15.10.2021 KR 20210137890
(71) Applicant: CJ CheilJedang Corporation, Seoul 04560 (KR)
(72) Inventor: CHANG, Jin Sook, Seoul 04560 (KR); KIM, Hyo Kyung, Seoul 04560 (KR); CHOI, Sun Hyoung, Seoul 04560 (KR); LEE, Zeewon, Seoul 04560 (KR)
(74) Representative: Regimbeau
(86) International application number: PCT/KR2022/015551
(87) International publication number: WO 2023/063763

(57) **Abstract**

The present disclosure pertains to: an L-arginine-producing microorganism in which proteins containing the amino acid sequence of SEQ ID NO: 1 are attenuated; and a method for producing L-arginine using same.

## Description

### [Technical Field]

The present disclosure relates to an L-arginine producing microorganism in which the activity of a protein including the amino acid sequence of SEQ ID NO: 1 is weakened and a method for producing L-arginine.

### [Background Art]

L-arginine is used for medicines, such as a liver function promoter, a brain function promoter, and a complex amino acid preparation, and has recently been receiving an attention for foods, such as a fish cake additive, a health drink additive, and a salt substitute for hypertensive patients. Research is continuously made to use microorganisms to produce industrially applicable arginine at high concentrations, and reports on methods of using mutant strains derived from microorganisms of the genus Brevibacterium or *Corynebacterium,* which are glutamate producing strains, and methods of using amino acid producing strains with improved growth through cell fusion, and the like have been made (US 8,034,602 B2).

### [Disclosure]

### [Technical Problem]

An aspect of the present disclosure is to provide a recombinant microorganism of the genus *Corynebacterium* producing L-arginine, a method for producing L-arginine by using the same, and use thereof.

### [Technical Solution]

In accordance with an aspect of the present disclosure, there is provided a recombinant microorganism of the genus *Corynebacterium* producing L-arginine, in which the activity of a protein including the amino acid sequence of SEQ ID NO: 1 is weakened.

In accordance with another aspect of the present disclosure, there is provided a method for producing L-arginine, the method including culturing, in a medium, a recombinant microorganism of the genus *Corynebacterium* in which the activity of a protein including the amino acid sequence of SEQ ID NO: 1 is weakened.

### [Advantageous Effects]

The microorganism of the genus *Corynebacterium* in which the activity of a protein including the amino acid sequence of SEQ ID NO: 1 is weakened of the present disclosure can produce L-arginine with high yield and thus can be advantageously used for industrial production.

### [Detailed Description of the Invention]

The present disclosure will be specifically described as follows. Each description and embodiment disclosed in the present disclosure may also be applied to other descriptions and embodiments. That is, all combinations of various elements disclosed in the present disclosure fall within the scope of the present disclosure. Furthermore, the scope of the present disclosure is not limited by the specific description below. Furthermore, throughout the overall specification, many papers and patent documents are referenced and their citations are provided. The disclosures of cited papers and patent documents are entirely incorporated by reference herein, and the level of the technical field within which the present invention falls and details of the present invention are explained more clearly.

In accordance with an aspect of the present disclosure, there is provided a recombinant microorganism of the genus *Corynebacterium* in which the activity of a protein including the amino acid sequence of SEQ ID NO: 1 is weakened.

The protein of the present disclosure may have or include the amino acid sequence set forth in SEQ ID NO: 1 or may consist of or essentially consist of the amino acid sequence.

In the present disclosure, the protein including the amino acid sequence of SEQ ID NO: 1 may be an endogenous protein of the microorganism of the present disclosure, but is not limited thereto.

The amino acid sequence of SEQ ID NO: 1 may be obtained from the U.S. National Institutes of Health Gene Bank (NIH GenBank), a known database. In the present disclosure, the amino acid sequence of SEQ ID NO: 1 may include an amino acid sequence having at least 70%, 75%, 80%, 85%, 90%, 95%, 96%, 97%, 98%, 99%, 99.5%, 99.7%, or 99.9% homology or identity to the amino acid sequence set forth in SEQ ID NO: 1. It would be obvious that any protein that has an amino acid sequence with deletion, modification, substitution, conservative substitution, or addition in a portion thereof may also be included within the range of the present disclosure as long as the amino acid sequence has such homology or identity and exhibits efficacy corresponding to the protein of the present disclosure.

For example, the amino acid sequence may include an amino acid sequence having a sequence addition or deletion, a naturally occurring mutation, a silent mutation, or a conservative substitution that does not alter functions of the protein of the present disclosure, at the N-terminus, C-terminus, and/or inside of the amino acid sequence.

As used herein, the term "conservative substitution" refers to a substitution of one amino acid with another amino acid that has similar structural and/or chemical properties. The protein may have, for example, one or more conservative substitutions while still retaining one or more biological activities. Such an amino acid substitution may be generally made on the basis of similarity in polarity, charge, solubility, hydrophobicity, hydrophilicity, and/or amphipathic nature of the residues. For example, among the electrically charged amino acids, positively charged (basic) amino acids include arginine, lysine, and histidine; and negatively charged (acidic) amino acids include glutamic acid and aspartic acid. Among the uncharged amino acids, nonpolar amino acids include glycine, alanine, valine, leucine, isoleucine, methionine, phenylalanine, tryptophan, and proline; polar or hydrophilic amino acids include serine, threonine, cysteine, tyrosine, asparagine, and glutamine. Among the amino acids, aromatic amino acids include phenylalanine, tryptophan, and tyrosine.

As used herein, the term "homology" or "identity" refers to the degree of similarity between two given amino acid sequences or nucleic acid base sequences, and may be expressed as a percentage. The terms homology and identity may be often used interchangeably with each other.

The sequence homology or identity of conserved polynucleotides or proteins may be determined by standard alignment algorithms, and default gap penalties established by a program to be used may be used together. Substantially, all or portions of homologous or identical sequences may generally hybridize with each other under moderate or highly stringent conditions. It would be obvious that the hybridization also includes hybridization with a polynucleotide containing usual codons or codons considering codon degeneracy in the polynucleotide.

The homology, similarity, or identity between any two polynucleotide or protein sequences may be determined using a known computer algorithm, such as "FASTA" program, by using default parameters as in the literature (Pearson et al., (1988) [Proc. Natl. Acad. Sci. USA 85]: 2444). Alternatively, this may be determined using the Needleman-Wunsch algorithm (Needleman and Wunsch, 1970, J. Mol. Biol. 48: 443-453), which is performed in the Needleman program of the European Molecular Biology Open Software Suite (EMBOSS) package (Rice et al., 2000, Trends Genet. 16: 276-277) (version 5.0.0 or versions thereafter) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.] (1988) SIAM J Applied Math 48: 1073). For example, the homology, similarity, or identity may be determined using BLAST of the National Center for Biotechnology Information database, or ClustalW

The homology, similarity, or identity between polynucleotides or proteins may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines similarity as the number of aligned symbols (i.e., nucleotides or amino acids) which are similar, divided by the total number of symbols in the shorter of the two sequences. The default parameters for the GAP program may include: (1) a binary comparison matrix (containing a value of 1 for identity and 0 for non-identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

In the present disclosure, a polynucleotide encoding the protein including the amino acid sequence of SEQ ID NO: 1 may be named NCgl1469 gene.

As used herein, the term "polynucleotide" refers to a polymer of nucleotides chain-extended lengthwise by covalent bonds of nucleotide monomers, which is a DNA or RNA strand with a certain length, and more specifically, a polynucleotide fragment encoding the protein.

The polynucleotide encoding the protein of the present disclosure may include a nucleic acid base sequence encoding the amino acid sequence set forth in SEQ ID NO: 1. As an example, the polynucleotide of the present disclosure may have or include the nucleic acid base sequence of SEQ ID NO: 2. Alternatively, the polynucleotide of the present disclosure may consist of or essentially consist of the nucleic acid base sequence of SEQ ID NO: 2.

In the polynucleotide of the present disclosure, various modifications may be made in the coding region as long as the amino acid sequence of the protein of the present disclosure is not changed in consideration of codon degeneracy or codons preferred in an organism that is intended to express the protein of the present disclosure. Specifically, the polynucleotide of the present disclosure may have or include a nucleic acid base sequence having at least 70%, at least 75%, at least 80%, at least 85%, at least 90%, at least 95%, at least 96%, at least 97%, or at least 98%, but less than 100% homology or identity to the nucleic acid sequence of SEQ ID NO: 2, or may consist of or essentially consist of a nucleotide sequence having at least 70%, at least 75%, at least 80%, at least 85%, at least 90%, at least 95%, at least 96%, at least 97%, or at least 98%, but less than 100% homology or identity to the nucleic acid base sequence of SEQ ID NO: 2, but is not limited thereto.

In addition, the polynucleotide of the present disclosure may include, without limitation, a sequence that can hydride, under stringent conditions, with a probe capable of being prepared from a known gene sequence, for example, a sequence complementary to a part of or the entire polynucleotide sequence of the present disclosure. The term "stringent conditions" refers to conditions that enable specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; and F. M. Ausubel et al., Current Protocols in Molecular Biology, John Wiley & Sons, Inc., New York, 9.50-9.51, 11.7-11.8). Examples of the stringent conditions may include conditions in which polynucleotides having higher homology or identity, for example, polynucleotides having at least 70%, at least 75%, at least 80%, at least 85%, at least 90%, at least 95%, at least 96%, at least 97%, at least 98%, or at least 99% homology or identity, hybridize with each other, but polynucleotides having lower homology or identity do not hybridize with each other; or conditions of washing of typical Southern hybridization, i.e., conditions of washing once, or two or three times, at a salt concentration and temperature corresponding to 1×SSC, 0.1% SDS at 60°C, specifically 0.1×SSC, 0.1% SDS at 60°C, or more specifically 0.1×SSC, 0.1% SDS at 68°C.
Hybridization requires that two nucleic acids have complementary sequences although mismatches between bases are possible depending on the stringency of the hybridization. The term "complementary" is used to describe the relationship between nucleotide bases that can hybridize with each another. For example, with respect to DNA, adenine is complementary to thymine and cytosine is complementary to guanine. Accordingly, the polynucleotide of the present disclosure may also include isolated nucleic acid fragments complementary to the complete sequence as well as substantially similar nucleic acid base sequences.

Specifically, polynucleotides having homology or identity to the polynucleotide of the present disclosure may be detected by employing hybridization conditions including a hybridization step at Tm of 55°C and utilizing the above-described conditions. In addition, the Tm value may be 60°C, 63°C, or 65°C, but is not limited thereto, and may be appropriately adjusted according to the purpose by a person skilled in the art.

The appropriate degree of stringency for hybridizing polynucleotides depends on the length of the polynucleotide and the degree of complementarity thereof, and variables thereof are well known in the art (e.g., Sambrook et al., supra).

As used herein, the term "microorganism (or strain)" encompasses all of wild-type microorganisms or naturally or artificially genetically modified microorganisms, refers to a microorganism in which a particular mechanism is diminished or enhanced due to the insertion of an exogenous gene or the enhancement or inactivation of the activity of an endogenous gene, and may be a microorganism including a target protein or a genetic modification for production of a protein or product.

The microorganism of the present disclosure may be a microorganism in which the protein including the amino acid sequence of SEQ ID NO: 1 of the present disclosure is weakened or the polynucleotide encoding the protein is deleted; or a microorganism that has been genetically modified (e.g., a recombinant microorganism) through a vector so as to weaken the protein including the amino acid sequence of SEQ ID NO: 1 of the present disclosure or delete the polynucleotide encoding the protein, but is not limited thereto.

The microorganism of the present disclosure may be a microorganism having an L-arginine producing ability.

The microorganism of the present disclosure may be a microorganism having an increased L-arginine producing ability compared with a parent strain.

The microorganism of the present disclosure may be a microorganism obtained by imparting an L-arginine producing ability to a parent strain (e.g., a parent strain naturally having an L-arginine producing ability or lacking an L-arginine producing ability) through the weakening of the protein including the amino acid sequence of SEQ ID NO: 1 of the present disclosure or the deletion of the polynucleotide encoding the protein, but is not limited thereto.

For example, the recombinant microorganism of the present disclosure may be a strain or microorganism in which the protein including the amino acid sequence of SEQ ID NO: 1 of the present disclosure is weakened or the polynucleotide encoding the protein is deleted, by transformation with a vector so as to weaken the protein including the amino acid sequence of SEQ ID NO: 1 of the present disclosure or delete the polynucleotide encoding the protein, wherein the protein including the amino acid sequence of SEQ ID NO: 1 is weakened or the polynucleotide encoding the protein is deleted in a native wild-type microorganism or an L-arginine producing microorganism, the L-arginine producing ability of the microorganism is increased compared with a native wild-type microorganism or a microorganism in which the protein including the amino acid sequence of SEQ ID NO: 1 or the polynucleotide encoding the protein is not modified, but is not limited thereto.

For example, a non-modified microorganism or parent strain as a comparison strain for comparing an increase in L-arginine production ability may be ATCC13869 strain or *Corynebacterium glutamicum* KCCM10741P (van der Rest et al., Appl. Microbiol Biotechnol 52:541-545, 1999) or a strain in which *argR* gene is deleted in wild-type *Corynebacterium glutamicum* ATCC13869 (e.g., *Corynebacterium glutamicum* CJ1R), but is not limited thereto.

As an example, the recombinant strain with increased producing ability may have an L-arginine producing ability, which is increased by at least about 1%, specifically, at least about 1%, at least about 2.5%, at least about 5%, at least about 6%, at least about 7%, at least about 8%, at least about 9%, at least about 10%, at least about 10.5%, at least about 11%, at least about 11.5%, at least about 12%, at least about 12.5%, at least about 13%, at least about 13.5%, or at least about 14% (the upper bound is not particularly limited, and the upper bound may be, for example, at most about 200%, at most about 150%, at most about 100%, at most about 50%, at most about 40%, at most about 30%, or at most about 25%), compared with that of the parent strain before modification or the non-modified microorganism, but the L-arginine producing ability is not limited thereto as long as it has an increment of a plus (+) value compared with the producing ability of a parent strain before modification or a non-modified microorganism. As another example, the recombinant strain with increased producing ability may have an L-arginine producing ability, which is increased by at least about 1.1 times, at least about 1.12 times, at least about 1.13 times, or at least about 1.14 times (the upper bound is not particularly limited, and the upper bound may be, for example, at most about 10 times, at most about 5 times, at most about 3 times, or at most about 2 times), compared with that of a parent strain before modification or a non-modified microorganism, but the L-arginine producing ability is not limited thereto. As used herein, the term "about" refers to a range encompassing all of ±0.5, ±0.4, ±0.3, ±0.2, ±0.1, and the like, and thus encompasses all of the values in the range equivalent or similar to those stated after this term, but is not limited thereto.

As used herein, the term "non-modified microorganism" may refer to a wild-type strain or a native strain as it is, or a strain before transformation due to the genetic mutation caused by natural or artificial factors, not excluding strains including mutations that may naturally occur in microorganisms. For example, the non-modified microorganism may mean a microorganism before the weakening of the protein including the amino acid sequence of SEQ ID NO: 1 herein or the deletion of the polynucleotide encoding the protein. The "non-modified microorganism" may be used interchangeably with "strain before modification", "microorganism before modification", "non-mutated strain", "non-modified strain", "non-mutated microorganism" or "reference microorganism".

As still another example of the present disclosure, the microorganism of the genus *Corynebacterium* of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Specifically, the recombinant microorganism of the present disclosure may be a microorganism in which all or a portion of the polynucleotide encoding the protein including the amino acid sequence of SEQ ID NO: 1 is deleted.

In addition, the microorganism of the present disclosure may be a microorganism in which the activity of an arginine repressor (ArgR) is additionally weakened. Specifically, the microorganism of the present disclosure may be a microorganism in which all or a portion of *argR* gene is additionally deleted.

ArgR may include a polypeptide set forth in the amino acid sequence of SEQ ID NO: 13. The *argR* gene may include a polynucleotide set forth in the nucleic acid base sequence of SEQ ID NO: 14, but is not limited thereto.

As an example, the microorganism of the present disclosure may be a strain in which NCgl1469 gene is deleted in the *Corynebacterium glutamicum* KCCM10741P (van der Rest et al., Appl. Microbiol Biotechnol 52:541-545, 1999) or a strain in which NCgl1469 gene is deleted in the *Corynebacterium glutamicum* CJ1R.

In addition, the microorganism of the present disclosure may include ornithine carbamoyltransferase subunit F (ArgF), a polynucleotide encoding the ArgF, or *argF* gene. The ArgF of the present disclosure may consist of the amino acid sequence set forth in SEQ ID NO: 15. The *argF* gene of the present disclosure may consist of the nucleic acid base sequence set forth in SEQ ID NO: 16.

As used herein, the term "weakening" of the protein has a concept encompassing all of the reduction of activity or the absence of activity compared with the intrinsic activity. The weakening may be used interchangeably with inactivation, deficiency, down-regulation, decrease, reduction, attenuation, or the like.

The weakening may also include: a case where the activity of the protein itself is reduced or eliminated compared with the activity of the protein possessed by an original microorganism due to the mutation or the like of the polynucleotide encoding the protein; a case where the entire activity and/or concentration (expression level) of the protein in a cell is lower compared with a native strain due to the inhibition of the expression of a gene of a polynucleotide encoding the protein or the inhibition of the translation into the protein; a case where the expression of the polynucleotide is not attained; and/or a case where the protein has no activity in spite of the expression of the polynucleotide. The term "intrinsic activity" refers to the activity of a specific protein originally possessed by a parental strain before modification or a wild-type or non-modified microorganism when a trait is changed due to genetic mutation caused by a natural or artificial factor. This term may be used interchangeably with the "activity before modification". The "inactivation", "deficiency", "decrease", "down-regulation", "reduction", or "attenuation" of the activity of a protein compared with the intrinsic activity thereof means that the activity of the protein is lowered compared with the activity of a specific protein originally possessed by a parent strain before transformation or a non-modified microorganism.

The weakening of the activity of the protein may be performed by any method known in the art, but is not limited thereto, and may be attained by applying various methods well known in the art (e.g., Nakashima N et al., Bacterial cellular engineering by genome editing and gene silencing. Int J Mol Sci. 2014;15(2):2773-2793; and Sambrook et al. Molecular Cloning 2012).

Specifically, the weakening of the protein of the present disclosure may be achieved by:
1) deleting all or a portion of the gene encoding the protein;
2) modifying an expression control region (or expression control sequence) so as to reduce the expression of the gene encoding the protein;
3) modifying the amino acid sequence (e.g., deletion/substitution/addition of at least one amino acid in the amino acid sequence) constituting the protein so as to eliminate or weaken the activity of the protein;
4) modifying the gene sequence encoding the protein so as to eliminate or weaken the activity of the protein (e.g., deletion/substitution/addition of at least one nucleic acid base in the nucleic acid base sequence of the protein gene so as to encode the protein modified to eliminate or weaken the activity of the protein);
5) modifying the nucleic acid base sequence encoding the initiation codon or 5'-UTR region of the transcript of the gene encoding the protein;
6) introducing an antisense oligonucleotide (e.g., antisense RNA) complementarily binding to the transcript of the gene encoding the polypeptide;
7) adding a sequence complementary to the Shine-Dalgarno sequence of the gene encoding the protein to the upstream of the Shine-Dalgarno sequence so as to form a secondary structure that makes the attachment of ribosomes impossible;
8) adding a promoter, which is to be reverse-transcribed, to the 3' end of the open reading frame (ORF) of the gene sequence encoding the protein (reverse transcription engineering, RTE); or
9) a combination of two or more selected from items 1) to 8) above, but is not particularly limited thereto.

For example, these are described as follows.

The deleting of all or a portion of the gene encoding the protein in item 1) above may be eliminating all of the polynucleotide encoding an endogenous target protein in the chromosome, replacing with a polynucleotide with deletions of some nucleotides, or replacing with a marker gene.

The modifying of an expression control region (or expression control sequence) in item 2) above may be inducing a mutation on the expression control region (or expression control sequence) through deletion, insertion, non-conservative or conservative substitution, or a combination thereof, or replacing with a sequence having a more weakened activity. The expression control region includes a promoter, an operator sequence, a sequence for encoding a ribosomal binding site, and a sequence for controlling the termination of transcription and translation, but is not limited thereto.

The modifying of the nucleic acid base sequence encoding the initiation codon or 5'-UTR region of the transcript of the gene encoding the protein in item 3) above may be, for example, substituting with a nucleic acid base sequence encoding, rather than an endogenous initiation codon, another initiation codon having a lower expression rate of the protein, but is not limited thereto.

The modifying of the amino acid sequence or polynucleotide sequence in items 4) and 5) above may be inducing a mutation on the sequence through deletion, insertion, non-conservative or conservative substitution, or a combination thereof in the amino acid sequence of the protein or the polynucleotide sequence encoding the protein so as to weaken the activity of the protein, or replacing with an amino acid sequence or polynucleotide sequence modified to have further weakened activity or an amino acid sequence or polynucleotide sequence modified to have no activity, but are not limited thereto. For example, the expression of the gene may be inhibited or weakened by introducing mutation into the polynucleotide sequence to form a termination codon.

The introducing of an antisense oligonucleotide (e.g., antisense RNA) complementarily binding to the transcript of the gene encoding the protein in item 6) above may be referred to, for example, the literature [Weintraub, H. et al., Antisense-RNA as a molecular tool for genetic analysis, Reviews - Trends in Genetics, Vol. 1(1) 1986].

The adding of a sequence complementary to the Shine-Dalgarno sequence of the gene encoding the protein to the upstream of the Shine-Dalgarno sequence so as to form a secondary structure that makes the attachment of ribosomes impossible in item 7) above may be making mRNA translation impossible or reducing the rate thereof.

The adding of a promoter, which is to be reverse-transcribed, to the 3' end of the open reading frame (ORF) of the gene sequence encoding the protein (reverse transcription engineering, RTE) in item 8) above may be making an antisense nucleotide complementary to the gene transcript encoding the polypeptide to thereby weaken the activity of the protein.

As used herein, the term "enhancement" of the activity of the protein refers to increasing of the activity of the protein compared with intrinsic activity thereof. The term enhancement may be used interchangeably with the terms, such as activation, up-regulation, overexpression, increase, and the like. Particularly, the activation, enhancement, up-regulation, overexpression, and increase may include exhibiting the activity originally possessed or exhibiting the activity that was improved compared with the intrinsic activity or activity before modification. The "intrinsic activity" refers to the activity of a specific protein that is originally possessed by a parental strain before transformation or a non-modified microorganism when a trait is changed due to genetic mutation caused by a natural or artificial factor. This term may be used interchangeably with the "activity before modification". The "enhancement", "up-regulation", "overexpression", or "increase" of the activity of a protein compared with the intrinsic activity thereof means that the activity and/or concentration (expression level) of a specific polypeptide originally possessed by a parent strain before transformation or a non-modified microorganism.

The enhancement may be achieved through the introduction of an exogenous protein or the increase of the activity and/or concentration (expression level) of an endogenous protein. The enhancement of the activity of the protein may be confirmed by an increase in the degree of activity or the expression level of the corresponding protein or an increase in the amount of a product excreted from the corresponding protein.

The enhancement of the activity of the protein may be attained by applying various methods well known in the art, and the methods are not limited as long as the methods can enhance the activity of a target protein compared with that of a microorganism before transformation. Specifically, the enhancement of the activity of the protein may be achieved by using genetic engineering and/or protein engineering well known to a person skilled in the art, which are routine methods in the molecular biology, but is not limited thereto (e.g., Sitnicka et al. Functional Analysis of Genes. Advances in Cell Biology. 2010, Vol. 2. 1-16; and Sambrook et al. Molecular Cloning 2012).

Specifically, the enhancement of the protein of the present disclosure may be achieved by:
1) increasing the intracellular copy number of a polynucleotide encoding the protein;
2) replacing the expression control region on the chromosome encoding the protein with a sequence with stronger activity;
3) modifying the nucleic acid base sequence encoding the initiation codon or 5'-UTR region of the gene transcript encoding the protein;
4) modifying the amino acid sequence of the protein so as to enhance the activity of the protein;
5) modifying the polynucleotide sequence encoding the protein so as to enhance the activity of the protein (e.g., modifying the polynucleotide sequence of the protein gene so as to encode a protein modified to enhance the activity of the protein);
6) introducing an exogenous protein exhibiting the activity of the protein or an exogenous polynucleotide encoding the same;
7) codon optimization of the polynucleotide encoding the protein;
8) modifying or chemical modifying an exposed site selected by analysis of a tertiary structure of the protein; or
9) a combination of two or more selected from items 1) to 8) above, but is not particularly limited thereto.

A description is made more specifically as follows.

The increasing of the intracellular copy number of the polynucleotide encoding the protein in item 1) above may be achieved by introducing, into a host cell, a vector operably linked to the polynucleotide encoding the corresponding protein and able to replicate and function regardless of a host. Alternatively, the increasing may be achieved by introducing at least one copy of polynucleotide encoding the corresponding protein into the chromosome of the host cell. The introduction into the chromosome may be performed by the introduction, into a host cell, of a vector capable of inserting the polynucleotide into the chromosome in the host cell, but is not limited thereto. The vector is as described above.

The replacing of the expression control region (or expression control sequence) on the chromosome encoding the protein with a sequence with stronger activity in item 2) above may be, for example, inducing a mutation on the sequence through deletion, insertion, non-conservative or conservative substitution, or a combination thereof to further enhance the activity of the expression control region, or replacing with a sequence having stronger activity. The expression control region may include, but is not particularly limited to, a promoter, an operator sequence, a sequence encoding a ribosome binding site, a sequence controlling the termination of transcription and translation, and the like. For example, the replacing may be replacing the original promoter with a strong promoter, but is not limited thereto.

Known examples of the stronger promoter may include CJ1 to CJ7 promoters (US 7662943 B2), lac promoter, trp promoter, trc promoter, tac promoter, lamda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but are not limited thereto.

The modifying of the nucleic acid base sequence encoding the initiation codon or 5'-UTR region of the gene transcript encoding the protein in item 3) above may be, for example, substituting with a nucleic acid base sequence encoding, rather than an intrinsic initiation codon, another initiation codon having a lower expression rate of the protein, but is not limited thereto.

The modifying of the amino acid sequence or polynucleotide sequence in items 4) and 5) above may be inducing a mutation on the sequence through deletion, insertion, non-conservative or conservative substitution, or a combination thereof in the amino acid sequence of the protein or the polynucleotide sequence encoding the protein so as to enhance the activity of the protein, or replacing with an amino acid sequence or polynucleotide sequence modified to further enhance the activity or an amino acid sequence or polynucleotide sequence modified to increase the activity of the protein, but are not limited thereto. The replacing may be performed by inserting the polynucleotide into the chromosome by homologous recombination, but is not limited thereto. The vector used herein may further include a selection maker for identifying the insertion into the chromosome. The selection marker is as described above.

The introducing of an exogenous polypeptide exhibiting the activity of the polypeptide in item 6) above may be introducing, into a host cell, an exogenous polynucleotide encoding a protein exhibiting the same/similar activity to the protein. The exogenous polynucleotide is not limited to the origin or sequence thereof as long as the exogenous polynucleotide exhibits the same/similar activity to the protein. The introduction may be performed by any known transformation method that is appropriately selected by a person skilled in the art, and the introduced polynucleotide is expressed in the host cell and thus the protein is produced and the activity thereof can be increased.

The codon optimization of the polynucleotide encoding the protein in item 7) may be the codon optimization of an endogenous polynucleotide so as to increase transcription or translation thereof in a host cell, or the codon optimization of an exogenous polynucleotide so as to perform optimized transcription or translation in a host cell.

The modifying or chemical modifying of an exposed site selected by analysis of a tertiary structure of the protein in item 8) above may be, for example, modifying or chemical modifying an exposed site to be modified or chemically modified by comparing sequence information of a protein to be analyzed, with a database that stores sequence information of existing proteins to determine a template protein candidate according to similarity of the sequences and identifying the structure on the basis of the determined candidate.

The enhancement of the activity of the protein may mean that the activity, concentration, or expression level of the corresponding protein is increased relative to the activity or concentration of the protein expressed in a wild-type microbial strain or a microbial strain before modification, or that the amount of a product produced from the corresponding polypeptide is increased, but is not limited thereto.

As used herein, the term "vector" refers to a DNA construct that includes a nucleic acid base sequence of the polynucleotide encoding the target protein and operably linked to a suitable expression control region (or expression control sequence) so that the target polypeptide can be expressed in an appropriate host. The expression control region may include a promoter capable of initiating transcription, any operator sequence for controlling such transcription, a sequence for encoding a suitable mRNA ribosomal binding site, and a sequence for controlling the termination of transcription and translation. The vector, after transformation into a suitable host cell, can replicate or function irrespective of the genome of the host, or may be integrated into the genome itself.

The vector used in the present disclosure is not particularly limited, and any vector known in the art may be used. Examples of the vector that is commonly used may include native or recombinant plasmids, cosmids, viruses, and bacteriophages. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescriptll-based, pGEM-based, pTZ-based, pCL-based, and pET-based vectors may be used as plasmid vectors. Specifically, pDZ, pDC, pDCM2, pACYC177, pACYC184, pCL, pECCG117, pUC19, pBR322, pMW118, and pCC1BAC vectors may be used.

As an example, a polynucleotide encoding a target polypeptide may be inserted in a chromosome through a vector for chromosomal insertion into a chromosome. The insertion of the polynucleotide into the chromosome may be performed using any method known in the art, for example, homologous recombination, but is not limited thereto. The vector may further include a selection maker for investigating the insertion or non-insertion into the chromosome. The selection marker is used for selecting cells transformed with the vector, that is, identifying the insertion or non-insertion of a target nucleic acid molecule, and markers imparting a selectable phenotype, such as drug resistance, auxotrophy, cytotoxic drug resistance, or surface polypeptide expression may be used. Under the circumstances of the treatment with selective agents, only cells expressing selection markers can survive or exhibit other phenotypic traits, thereby enabling the selection of transformed cells.

As used herein, the term "transformation" means that a vector containing a polynucleotide encoding a target polypeptide is introduced into a host cell or microorganism to allow the polypeptide encoded by the polynucleotide to be expressed in the host cell. Examples of the transformed polynucleotide may include any polypeptide as long as it can be expressed in a host cell, regardless of whether it is inserted and located into the chromosome of the host cell or located outside of the chromosome. In addition, the polynucleotide includes DNA and/or RNA encoding the target polypeptide. The polynucleotide may be introduced in any form as long as the polynucleotide can be introduced and expressed in a host cell. For example, the polynucleotide may be introduced in the form of an expression cassette, which is a gene construct containing all factors required for self-expression. The expression cassette may generally include a promoter operably linked to the polynucleotide, a transcription termination signal, a ribosome binding site, and a translation terminal signal. The expression cassette may be in the form of a self-replicable expression vector. In addition, the polynucleotide may be introduced as it is into the host cell to be operably linked to the sequence required for expression in the host cell, but is not limited thereto.

The term "operably linked" above refers to a functional linkage between a promoter sequence for initiating and mediating the transcription of the polynucleotide encoding the target protein of the present disclosure and the polynucleotide sequence.

In the microorganism of the present disclosure, the modification of all or a portion of the polynucleotide may be induced by (a) homologous recombination using a vector for chromosomal insertion into a chromosome or genome editing using an engineered nuclease (e.g., CRISPR-Cas9) and/or (b) treatment with light, such as UV rays and radioactive rays, and/or chemicals, without being limited thereto. The modifying of all or a portion of the gene may include a method using DNA recombinant technology. For example, all or a portion of the gene may be deleted by injecting a nucleotide sequence or vector, which contains a nucleotide sequence homologous to a target gene, into the microorganism to induce homologous recombination. The injected nucleotide sequence or vector may contain a dominant selection marker, but is not limited thereto.

In accordance with still another aspect of the present disclosure, there is provided a method for producing L-arginine, the method including culturing, in a medium, a recombinant microorganism of the genus *Corynebacterium* in which the activity of a protein including the amino acid sequence of SEQ ID NO: 1 is weakened.

The protein including the amino acid sequence of SEQ ID NO: 1, weakening, microorganism, and the like are as described in the other aspects.

The microorganism of the genus *Corynebacterium* may be *Corynebacterium glutamicum,* but is not limited thereto.

In the microorganism, the activity of an arginine repressor (ArgR) may be additionally weakened or *argR* gene may be additionally deleted, but is not limited thereto. The weakening and deletion are as described in the other aspects.

As used herein the term "culturing" refers to growing the microorganism of the genus *Corynebacterium* of the present disclosure in appropriately adjusted environment conditions. The culturing step of the present disclosure may be performed according to appropriate media or culture conditions known in the art. The culturing step may be easily adjusted, according to the selected strain, by a person skilled in the art. Specifically, the culturing may be performed in a batch type, a continuous type, and/or a fed-batch type, but is not limited thereto.

As used herein, the term "medium" refers to a mixture containing, as main ingredients, nutrient materials required for culturing the microorganism of the present disclosure, wherein the medium supplies nutrient materials, growth factors, and the like, including water essential for survival and growth. Specifically, the medium and other culture conditions for culturing the microorganism of the genus *Corynebacterium* of the present disclosure may be any medium that is used for common culturing microorganisms without particular limitation. However, the microorganism of the genus *Corynebacterium* of the present disclosure may be cultured in a common medium containing appropriate carbon sources, nitrogen sources, phosphorus sources, inorganic compounds, amino acids, and/or vitamins, under aerobic conditions, while the temperature, pH, and the like are adjusted.

Specifically, culture media for the microorganism of the genus *Corynebacterium* may be found in the literature ["Manual of Methods for General Bacteriology" by the American Society for Bacteriology (Washington D.C., USA, 1981)].

In the present disclosure, examples of the carbon sources may include: carbohydrates, such as glucose, saccharose, lactose, fructose, sucrose, and maltose; sugar alcohols, such as mannitol and sorbitol; organic acids, such as pyruvic acid, lactic acid, and citric acid; and amino acids, such as glutamic acid, methionine, and lysine. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of other carbon sources may be used without limitation. These carbon sources may be used alone or in combination of two or more, but is not limited thereto.

Examples of the nitrogen sources may include: inorganic nitrogen sources, such as ammonia, ammonium sulfate, ammonium chloride, ammonium acetate, ammonium phosphate, ammonium carbonate, and ammonium nitrate; amino acids, such as glutamic acid, methionine, and glutamine; and organic nitrogen sources, such as peptone, NZ-amine, meat extracts, yeast extracts, malt extracts, corn steep liquor, casein hydrolysates, fishes or decomposition products thereof, defatted soybean cake or decomposition products thereof. These nitrogen sources may be used alone or in combination of two or more thereof, but are not limited thereto.

Examples of the phosphorus sources may include potassium phosphate monobasic, potassium phosphate dibasic, and sodium-containing salts corresponding thereto. Examples of the inorganic compounds may include sodium chloride, calcium chloride, iron chloride, magnesium sulfate, iron sulfate, manganese sulfate, calcium carbonate, and the like, and besides, may include amino acids, vitamins, and/or suitable precursors. These constituent ingredients or precursors may be added to the medium in a batch or continuous manner. However, examples of the phosphorus sources are not limited thereto.

The pH of the medium may be adjusted by adding compounds, such as ammonium hydroxide, potassium hydroxide, ammonia, phosphoric acid, and sulfuric acid, to the medium in an appropriate manner during culturing of the microorganism of the genus *Corynebacterium* of the present disclosure. In addition, an anti-foaming agent, such as a fatty acid polyglycol ester, may be added to suppress foam formation during culturing. Additionally, oxygen or oxygen-containing gas may be injected into the medium to maintain the aerobic state of the medium, or nitrogen, hydrogen or carbon dioxide gas may be injected, without the injection of gas, to maintain the anaerobic or non-aerobic state of the medium, but is not limited thereto.

In the present disclosure, the temperature for culturing may be maintained at 20°C to 45°C, and specifically 25°C to 40°C, and culturing may be performed for about 10 hours to 160 hours, but is not limited thereto.

L-arginine produced by the culturing of the present disclosure may be released into the medium or may remain in cells.

The method for producing L-arginine of the present disclosure may further include preparing the microorganism of the genus *Corynebacterium* of the present disclosure, preparing the medium for culturing the microorganism, or a combination of these steps (regardless of the order, in any order), for example, before the culturing step.

The method for producing L-arginine of the present disclosure may further include recovering L-arginine from the medium resulting from the culturing (a medium where culturing has been performed) or the microorganism of the genus *Corynebacterium* in the medium resulting the culturing. The method of the present disclosure may further include the recovering step after the culturing step.

The recovering may be collecting desired L-arginine by using an appropriate method known in the art according to the method for culturing the microorganism of the present disclosure, for example, a batch, continuous, or fed-batch type of culturing. Examples of the method may include centrifugation, filtration, treatment with a crystallized protein precipitating agent (salting-out), extraction, ultrasonic disruption, ultra-filtration, dialysis, various types of chromatography, such as molecular sieve chromatography (gel filtration), adsorption chromatography, ion exchange chromatography, and affinity chromatography, HPLC, and a combination of these methods, and desired L-arginine can be recovered from the medium or microorganism by using an appropriate method known in the art.

The method for producing L-arginine of the present disclosure may further include a purification step. The purification may be performed by using an appropriate method known in the art. In an example, when the method for producing L-arginine of the present disclosure includes both the recovery step and the purification step, the recovery step and the purification step may be performed continuously or discontinuously regardless of the order, or may be performed simultaneously or integrated into one step, but is not limited thereto.

In accordance with still another aspect of the present disclosure, there is provided a composition for producing L-arginine, the composition containing: the microorganism of the genus *Corynebacterium* in which the activity of a protein including the amino acid sequence of SEQ ID NO: 1 is weakened; a medium in which the microorganism has been cultured, or a combination thereof.

The composition of the present disclosure may further contain any appropriate excipient that is commonly used in compositions for producing amino acids, and examples of the excipient may include a preserving agent, a wetting agent, a dispersing agent, a suspending agent, a buffer, a stabilizing agent, or an isotonic agent, but are not limited thereto.

In the composition of the present disclosure, the protein including the amino acid sequence of SEQ ID NO: 1, weakening, microorganism, culturing, medium, and the like are as described in the other aspects.

In accordance with still another aspect of the present disclosure, there is provided a method for preparing a microorganism of the genus *Corynebacterium,* the method including weakening the activity of a protein including the amino acid sequence of SEQ ID NO: 1.

In accordance with still another aspect of the present disclosure, there is provided use, for producing L-arginine, of a microorganism of the genus *Corynebacterium* in which the activity of a protein including the amino acid sequence of SEQ ID NO: 1 is weakened.

In the method for preparing the microorganism of the genus *Corynebacterium* and use of the microorganism of the genus *Corynebacterium* for producing L-arginine, the protein including the amino acid sequence of SEQ ID NO: 1, weakening, microorganism, and the like are as described in the other aspects.

### [Mode for Carrying Out the Invention]

Hereinafter, the present disclosure will be described in detail with reference to examples. However, these examples are merely preferable embodiments for illustrating the present disclosure, and therefore are not intended to limit the scope of right of the present disclosure. Meanwhile, the technical matters not described herein can be sufficiently understood and easily implemented by a person skilled in the art or similar technical fields of the present disclosure.

### Example 1: Random mutant library construction using transposon and screening

To obtain strains having increased L-arginine producing ability, *Corynebacterium glutamicum* KCCM10741P (US 8034602 B2) as a parent strain was transformed with a plasmid, obtained using the EZ-Tn5^{™} <R6Kγori/KAN-2>Tnp Transposome^{™} Kit (Epicentre), by an electric pulse method (Appl. Microbiol. Biotechnol. (1999) 52:541-545), and then the transformants were plated on a complex plate medium containing kanamycin (25 mg/L), thereby obtaining about 20,000 colonies.

### <Complex plate medium (pH 7.0)>

Glucose 10 g, Peptone 10 g, Beef Extract 5 g, Yeast Extract 5 g, Brain Heart Infusion 18.5 g, NaCl 2.5 g, Urea 2 g, Sorbitol 91 g, Agar 20 g (based on 1 liter of distilled water)

The top seven mutant strains with improved L-arginine producing ability compared with *Corynebacterium glutamicum* KCCM10741P strain as the parent strain were selected by using the ninhydrin method (Moore, S., Stein, W. H., Photometric ninhydrin method for use in the chromatography of amino acids. J. Biol. Chem. 1948, 176, 367-388).

### Example 2: Analysis of L-arginine producing ability of selected random mutant strains

To eventually select strains with reproducibly increased L-arginine producing ability from the seven mutant strains selected in Example 1, flask culture was performed using the following medium. After the culture was completed, the concentration of L-arginine in the culture was analyzed by HPLC. The concentrations of L-arginine produced by each of the mutant strains are shown in Table 1 below.

### <Production medium (pH 7.2)>

Glucose 60 g, Ammonium Sulfate 45 g, Magnesium Sulfate Heptahydrate 2 g, Potassium Phosphate Monobasic 2 g, Ammonium Chloride 10 g, Biotin 0.01 mg, Thiamine-HCl 0.1 mg, Calcium Pantothenate 2 mg, Nicotinamide 3 mg, Ferrous Sulfate 10 mg, Manganese Sulfate 10 mg, Zinc Sulfate 0.02 mg, Copper Sulfate 0.5 mg, Calcium Carbonate 30 g (based on 1 liter of distilled water).

**TABLE 1**

| Concentrations of L-arginine produced by seven selected random mutant strains | | | | | |
|---|---|---|---|---|---|
| | Strain | L-arginine (g/L) | | | |
| | | Batch 1 | Batch 2 | Batch 3 | Mean |
| Control | KCCM10741P | 3 | 3.1 | 3.3 | 3.13 |
| 1 | KCCM10741P/mt-1 | 3.1 | 3 | 3.1 | 3.07 |
| 2 | KCCM10741P/mt-2 | 3 | 2.9 | 3 | 2.97 |
| 3 | KCCM10741P/mt-3 | 3.1 | 3 | 3.1 | 3.07 |
| 4 | KCCM10741P/mt-4 | 2.9 | 3 | 3.2 | 3.03 |
| 5 | KCCM10741P/mt-5 | 3.1 | 3.3 | 2.8 | 3.07 |
| 6 | KCCM10741P/mt-6 | 2.9 | 3.1 | 3.2 | 3.07 |
| 7 | KCCM10741P/mt-7 | 3.4 | 3.7 | 3.8 | 3.63 |

Among the ten selected mutant strains, KCCM10741P/mt-7 was eventually selected as a strain with significantly improved L-arginine producing ability.

### Example 3: Establishment of causes of increased L-arginine producing ability of eventually selected strain

A gene inactivated by random insertion of a transposon in KCCM10741P/mt-7 of Example 2 was identified.

Specifically, genomic DNA was extracted from KCCM10741P/mt-7, digested, and then ligated, and the ligation product was transformed into *E*. *coli* DH5α. The transformants were plated on an LB solid medium containing kanamycin (25 mg/L). After 20 transformed colonies were selected, plasmids containing an unknown gene portion were obtained, and the nucleic acid base sequences thereof were analyzed using primer 1 (SEQ ID NO: 3) and primer 2 (SEQ ID NO: 4) of the EZ-Tn5^{™} <R6Kγori/KAN-2> Tnp Transposome^{™} Kit. As a result, it was identified that a gene including the nucleotide sequence of SEQ ID NO: 2 was inactivated on the basis of the nucleic acid base sequence reported in the NIH GenBank of the U.S. National Institutes of Health.

**TABLE 2**

| Primer | Nucleic acid base sequence |
|---|---|
| Primer 1 (SEQ ID NO: 3) | ACCTACAACAAAGCTCTCATCAACC |
| Primer 2 (SEQ ID NO: 4) | CTACCCTGTGGAACACCTACATCT |

As a result of blast search of SEQ ID NO. 2 in the wild-type *Corynebacterium glutamicum* ATCC 13869, this sequence was identified to be NCgl1469 gene.

### Example 4: Construction of recombinant vector for NCgl1469 gene Deletion

To re-identify the influence of the inactivation of NCgl1469 gene on the production of L-arginine, a recombinant vector for deleting NCgl1469 gene on the chromosome of a strain of the genus *Corynebacterium* was constructed.

To manufacture fragments for deletion of the gene, primers 3 to 6 were synthesized, and the primers are shown in Table 3.

**TABLE 3**

| Primer | Nucleic acid base sequence |
|---|---|
| Primer 3 (SEQ ID NO: 5) | TCGAGCTCGGTACCC TCAGTGCTCCAACTGCTTG |
| Primer 4 (SEQ ID NO: 6) | CTTCAGGGGGATACCAGAATAGCGAAATGAAAAG |
| Primer 5 (SEQ ID NO: 7) | CTGGTATCCCCCTGAAGGCGATTTAAGGGGTCGA |
| Primer 6 (SEQ ID NO: 8) | CTCTAGAGGATCCCC GCAGGGGAGTTTGAAAAAC |

To construct a vector having a deletion of an ORF region on the basis of SEQ ID NO: 2, PCR was performed using wild-type *Corynebacterium glutamicum* ATCC13869 gDNA as a template along with a pair of primers of SEQ ID NOS: 5 and 6 and a pair of primers of SEQ ID NOS: 7 and 8. PCR was performed under conditions of denaturation at 95°C for 5 minutes, 30 cycles of denaturation at 95°C for 30 seconds, annealing at 55°C for 30 seconds, and polymerization at 72°C for 30 seconds, and then polymerization at 72°C for 5 minutes. Overlapping PCR was again performed using a mixture of two fragments obtained above as a temperate along with a pair of primers of SEQ ID NOS: 5 and 8, thereby obtaining fragments. PCR was performed under conditions of denaturation at 94°C for 5 minutes, 30 cycles of denaturation at 95°C for 30 seconds, annealing at 55°C for 30 seconds, and polymerization at 72°C for 1 minute, and then polymerization at 72°C for 5 minutes. pDCM2 vector (KR 10-2020-0136813) was treated with Smal, and the PCR products thus obtained were subjected to fusion cloning. Fusion cloning was performed using the In-Fusion^{®} HD cloning Kit (Clontech). The plasmid obtained from the cloning was named pDCM2-△NCgl1469.

### Example 5: Preparation of NCgl1469 gene-deleted strain and evaluation of L-arginine producing ability - 1

The *Corynebacterium glutamicum* KCCM10741P, which is an L-arginine producing strain, was transformed with pDCM2-△NCgl1469 by homologous recombination on the chromosome (van der Rest et al., Appl. Microbiol Biotechnol 52:541-545, 1999).

Thereafter, the transformants were subjected to secondary recombination on a solid medium plate containing 4% sucrose. The *Corynebacterium glutamicum* transformed strains after the completion of the secondary recombination were subjected to PCR using primers 3 and 6 to identify a strain in which the gene of SEQ ID NO: 2 was deleted. The recombinant strain was named *Corynebacterium glutamicum* KCCM10741P-△NCgl1469.

To analyze the L-arginine producing ability of the prepared *Corynebacterium glutamicum* KCCM10741P-△NCgl1469, the strain was cultured together with the parent strain *Corynebacterium glutamicum* KCCM10741P by the following method.

The parent strain *Corynebacterium glutamicum* KCCM10741P and the *Corynebacterium glutamicum* KCCM10741P-△NCgl1469 each was inoculated in a 250-mL corner-baffled flask containing 25 mL of the following seed medium and cultured with shaking at 200 rpm at 30°C for 20 hours. Then, 1 mL of the seed culture was inoculated in a 250-mL corner-baffle flask containing 24 mL of a production medium and cultured with shaking at 200 rpm at 30°C for 72 hours. The compositions of the seed medium and production medium were as follows.

### <Seed medium (pH 7.2)>

Glucose 20 g, Ammonium Sulfate 45 g, Magnesium Sulfate Heptahydrate 2 g, Potassium Phosphate Monobasic 2 g, Ammonium Chloride 10 g, Biotin 0.01 mg, Thiamine-HCl 0.1 mg, Calcium Pantothenate 2 mg, Nicotinamide 3 mg, Ferrous Sulfate 10 mg, Manganese Sulfate 10 mg, Zinc Sulfate 0.02 mg, Copper Sulfate 0.5 mg (based on 1 liter of distilled water).

### <Production medium (pH 7.2)>

Glucose 60 g, Ammonium Sulfate 45 g, Magnesium Sulfate Heptahydrate 2 g, Potassium Phosphate Monobasic 2 g, Ammonium Chloride 10 g, Biotin 0.01 mg, Thiamine-HCl 0.1 mg, Calcium Pantothenate 2 mg, Nicotinamide 3 mg, Ferrous Sulfate 10 mg, Manganese Sulfate 10 mg, Zinc Sulfate 0.02 mg, Copper Sulfate 0.5 mg, Calcium Carbonate 30 g (based on 1 liter of distilled water).

After the completion of the culture, the L-arginine producing ability was determined HPLC (Waters 2478), and the results are shown in Table 4 below.

**TABLE 4**

| | Strain | L-arginine (g/L) | | | |
|---|---|---|---|---|---|
| | | Batch 1 | Batch 2 | Batch 3 | Mean |
| Control | KCCM10741P | 2.9 | 3.0 | 3.05 | 2.98 |
| Test group | KCCM10741P-ΔNCgl1469 | 3.5 | 3.4 | 3.8 | 3.57 |

As a result, the KCCM10741P-△NCgl1469 showed a mean increase of 19.8% in L-arginine producing ability compared with the parent strain.

### Example 6: Preparation of NCgl1469 gene-deleted strain and evaluation of L-arginine producing ability - 2

It was identified whether another *Corynebacterium glutamicum* strain producing L-arginine also exhibited the same effect as in Example 5.

Specifically, a strain producing L-arginine was prepared by introducing one type of mutation (*argR* gene deletion, hereinafter △argR) into a wild-type *Corynebacterium glutamicum* strain (ATCC13869).

A recombinant vector for △argR was constructed by the same method as in the vector construction method of Example 4, and primers used for the construction of the vector are shown in Table 5.

**TABLE 5**

| Primer | Nucleic acid base sequence |
|---|---|
| Primer 7 (SEQ ID NO: 9) | TCGAGCTCGGTACCC AGCAGGCCTTAAGGGTAAG |
| Primer 8 (SEQ ID NO: 10) | AGGGGCGCTGTCTTACCTCGGCTGGTGGGCCAGC |
| Primer 9 (SEQ ID NO: 11) | GGTAAGACAGCGCCCCTAGTTCAAGGCTTGTTAATC |
| Primer 10 (SEQ ID NO: 12) | CTCTAGAGGATCCCC ACCGTTGAACTGCTTGCCAG |

A plasmid into which the target gene was inserted was selected by PCR, and then the plasmid was named pDCM2-△argR. The *Corynebacterium glutamicum* wild-type strain ATCC13869 was transformed with pDCM2-△argR by the same method as in Example 5. The transformant strains were subjected to PCR using primers 7 and 10 to identify a strain in which argR was deleted on the chromosome, and the strain was named *Corynebacterium glutamicum* CJ1R. The *Corynebacterium glutamicum* CJ1R was allowed to undergo the same method as in Example 5 to prepare a strain in which the NCgl1469 gene was deleted, and the strain was named CJ1R-△NCgl1469.

CJ1R-△NCgl1469 was cultured by the same method as in Example 5. After the completion of the culture, L-arginine producing ability was determined by HPLC (Waters 2478), and the results are shown in Table 6 below.

**TABLE 6**

| | Strain | L-arginine (g/L) | | | |
|---|---|---|---|---|---|
| | | Batch 1 | Batch 2 | Batch 3 | Mean |
| Control | CJ1R | 1.5 | 1.45 | 1.6 | 1.52 |
| Test group | CJ1R-ΔNCgl1469 | 1.85 | 1.72 | 1.9 | 1.82 |

As a result, the CJ1R-△NCgl1469 showed a mean increase of 19.7% in L-arginine producing ability compared with the parent strain CJ1R.

As set forth above, a person skilled in the art to which the present disclosure pertains will be able to understand that the present disclosure may be embodied in other specific forms without departing from the technical spirit or essential characteristics thereof. Therefore, the embodiments described above should be construed as being exemplified and not limiting the present specification. The scope of the present disclosure should be understood that all changes or modifications derived from the definitions and scopes of the claims and their equivalents fall within the scope of the application.

## Claims

1. A recombinant microorganism of the genus *Corynebacterium* producing L-arginine, in which the activity of a protein including the amino acid sequence of SEQ ID NO: 1 is weakened.

2. The microorganism of claim 1, wherein the microorganism has an increased L-arginine producing ability compared with a parent strain in which the activity of the protein including the amino acid sequence of SEQ ID NO: 1 is not weakened.

3. The microorganism of claim 1, wherein the microorganism is *Corynebacterium glutamicum.*

4. The microorganism of claim 1, wherein the activity of an arginine repressor is additionally weakened.

5. The microorganism of claim 1, wherein a polynucleotide encoding the protein including the amino acid sequence of SEQ ID NO: 1 is deleted.

6. A method for producing L-arginine, the method comprising culturing, in a medium, a recombinant microorganism of the genus *Corynebacterium* in which the activity of a protein including the amino acid sequence of SEQ ID NO: 1 is weakened.

7. The method of claim 6, wherein the microorganism is *Corynebacterium glutamicum.*

8. The method of claim 6, wherein in the microorganism of the genus *Corynebacterium,* the activity of an arginine repressor is additionally weakened.

9. The method of claim 6, wherein in the microorganism, a nucleic acid base sequence encoding the protein including the amino acid sequence of SEQ ID NO: 1 is deleted.

10. Use of the microorganism of any one of claims 1 to 5 for producing L-arginine.
